# EUROPEAN PATENT APPLICATION

(11) **EP 3 069 602 A1**
(43) Date of publication of application: **21.09.2016**
(21) Application number: 15000790.4
(22) Date of filing: 17.03.2015
(51) Int. Cl.: A01G 33/00, B01F 3/04, C12M 1/00, C12N 1/12, C02F 1/20, C02F 3/32

(54) **METHOD FOR DISSOLVING CO2 AND STRIPPING O2 BY USING A PERFORATED HOSE WITH LOW PRESSURE IN ALGAL PONDS**

(71) Applicant: Linde Aktiengesellschaft, 80331 München (DE)
(72) Inventor: van de Ven, Joost, 5235 DC 's-Hertogenbosch (NL)
(74) Representative: Gellner, Bernd

(57) **Abstract**

The present inventions relates to a method for solving carbon dioxide in an aqueous medium comprised within a tank, wherein gaseous carbon dioxide is discharged into said aqueous medium via at least one perforated hose. According to the invention, it is envisaged that the carbon dioxide is guided through the perforated hose with a gas flow of 0.05 to 0.2 kg CO₂/(m*h).

## Description

The invention relates to a method for solving CO₂ in an aqueous medium that is arranged in a tank.

Such a method comprises discharging gaseous carbon dioxide into the aqueous media via at least one perforated hose.

Biomass can be produced by variety of methods and means. One possibility for producing biomass is the breeding of algae in algae farms, wherein algae are cultivated in open ponds. The algae need, besides light and nutrients, especially carbon dioxide for maintaining life sustaining processes and particularly for growth, wherein carbon dioxide is converted into carbon hydrates in the end. Thus, an efficient supplementation of solved carbon dioxide would be desirable.

Furthermore, algae produce considerable amounts of oxygen by photosynthesis during their life and growth cycles. The production rate of oxygen may exceed the amount of oxygen that can be evaporated by the cultivation medium, so that unfavourable oxygen levels in the cultivation medium may be reached. Hence, it would be further desirable to provide simple and efficient means for reducing the oxygen level in the cultivation medium to an optimal value.

Therefore, the problem underlying the present invention is to provide an efficient and economic means for providing solved carbon dioxide in an aqueous medium, particularly in the context of algae production, and further for stripping excess oxygen from the aqueous medium.

This problem is solved in that the carbon dioxide is guided through the at least one perforated hose with a gas flow of 0.05 to 0.2 kg CO₂ per meter of the hose and per hour (also denoted as 0.05 to 0.2 kg CO₂/(m*h)).

Particularly, the at least one perforated hose is made of chemical resistant elastomer or comprises the latter. When the carbon dioxide is switched on, the pores open and small bubbles of gas are forming and emitted into the aqueous medium. The carbon dioxide is particularly injected without requiring an additional energy source.

Advantageously, carbon dioxide is dissolved in the aqueous medium with high efficiency by the method of the invention. Particularly, the distribution of carbon dioxide in the aqueous medium is improved by the method of the invention such that the solving efficiency is increased and a more constant pH in the aqueous medium is achievable. The method of the invention is particularly suitable for solving or discharging carbon dioxide in shallow aqueous media, particularly characterized by a depth of 0.10 m to 0.40 m. Particularly, the usage of a low gas flow causes the forming gas bubbles comprising carbon dioxide to stay long on the hose before going up to the surface. Oxygen, which is for example produced by the algae, can enter the aforementioned gas bubbles. The gas bubbles then contain high concentrations of oxygen as they go up to the surface. As a consequence, oxygen is efficiently stripped out of the aqueous medium.

As another advantage of the invention, there is no need for a sump in the tank. With normal injection systems gas bubbles raise from the injector (perforated hoses or other). In the time till the gas bubble reaches the surface the gas has time to solve. Therefore typically a sump with a higher depth is used providing the gas bubbles more time to solve. However, the use of sumps is accompanied by several disadvantages such as lowering the flow velocity of the aqueous medium, whereby particles like sand, algae or precipitates settle at the bottom of the tank causing problems with solving carbon dioxide. As described above, CO₂ is advantageously discharged into the aqueous medium by the method of the invention such that CO₂ in the bubble will solve more or less as fast as the gas comes out of the hose, so the gas bubbles stay long on the hose surface before they raise up in the water.

Particularly, the dosing of the carbon dioxide is controlled by a pH measurement.

In some embodiments, the carbon dioxide is guided through the perforated hose with an excess pressure of 0.1 to 1 bar with respect to the pressure of the aqueous medium. Advantageously, CO₂ needs to be provided with only a low pressure.

In some embodiments, the at least one perforated hose is at least partially arranged at the bottom of the tank.

In some embodiments, the at least one perforated hose is characterized by 1000 to 3000 pores per m [meter] of the hose, particularly with a pore size of 0.4 mm to 0.65 mm. In some embodiments, the perforated hose has an inner diameter of 10 mm to 19 mm, and particularly a wall thickness of 3 mm to 4.5 mm.

In some embodiments, the at least one perforated hose is made of an elastomer or comprises the latter. In some embodiments, the at least one hose is made of or comprises an ethylene-propylene elastomer or a styrene-butadiene elastomer, particularly, an ethylene-propylene diene rubber or a styrene-butadiene rubber.

In some embodiments, the tank is characterized by a fill level in the range from 0.1 m to 0.4 m.

In some embodiments, the at least one perforated hose is designed as a gas diffuser mat. In some embodiments, the carbon dioxide is discharged via a multitude of perforated hoses. In some embodiments, the multitude of perforated hoses is designed as a gas diffuser mat. The term "gas diffuser mat" in the context of the present invention refers to a flat structure that is formed by the at least on perforated hose or a multitude of perforated hoses. Advantageously, carbon dioxide can be more uniformly injected into the aqueous medium by use of such an arrangement.

In some embodiments, the gas diffuser mat is arranged at the bottom of the tank. In some embodiments, the gas diffuser mat is fixed at the bottom of the tank.

In some embodiments, the tank is an open pond or basin. In some embodiments, the tank is a raceway pond. Advantageously, the stripped oxygen can simply be emitted into the environment in such an open pond or basin.

In some embodiments, the tank is used for propagating a carbon dioxide assimilating organism, particularly an algae or cyanobacterium.

According to a further aspect of the invention, a method for propagating an autotrophic carbon dioxide assimilating organism is provided. The method comprises the steps of:
- providing a tank comprising an aqueous medium, wherein the aqueous medium comprises an autotrophic carbon dioxide assimilating organism,
- discharging a gas stream that comprises carbon dioxide into the aqueous medium, and
- cultivating the carbon dioxide assimilating organisms,
wherein the gas stream is discharged into the aqueous medium by the method for solving carbon dioxide in an aqueous medium comprised within a tank of the invention, particularly via at least one perforated hose, wherein the carbon dioxide gas steam is guided through the at least one perforated hose with a gas flow of 0.05 to 0.2 kg CO₂/(m*h), and particularly with an excess pressure of 0.1 to 1 bar with respect to the pressure of the aqueous medium.

In some embodiments, the carbon dioxide assimilating organism is an algae or a cyanobacterium.

In some embodiments, the carbon dioxide comprising gas stream is an exhaust stream from a combustion process, wherein a carbon containing compound is oxidized with air or oxygen.

In some embodiments, the carbon dioxide stream is provided by a carbon dioxide supply.

By using the method of the invention for solving carbon dioxide in an aqueous medium, the aqueous medium is efficiently supplemented with carbon dioxide. Simultaneously, when cultivating algae or cyanobacteria, excess oxygen that is produced by the aforementioned organisms can simply be stripped out of the aqueous medium before unfavorable amounts of O₂ can accumulate in the medium.

In some embodiments, the tank is an open pond. In some embodiments, the tank is a raceway pond. Advantageously, by using such an open pond, natural light can be used for cultivating the aforementioned algae or cyanobacteria, and the stripped oxygen can simply be emitted into the environment.

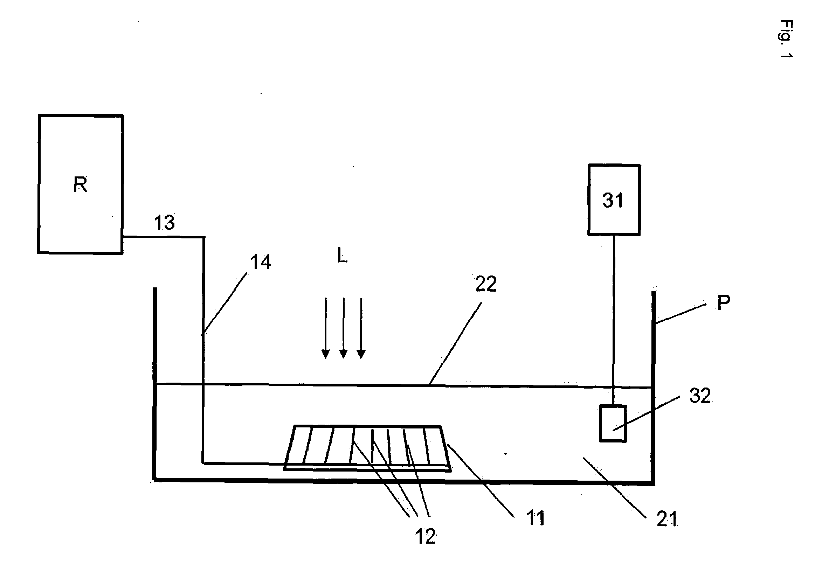

Further advantages, features and examples of the present invention shall be described in the following with reference to the Figure, wherein
- Fig. 1: shows a schematic illustration of an embodiment of the invention.

### Examples:

Fig. 1 shows a preferred embodiment of the invention, wherein a tank P is used for breeding algae. The tank P is designed as a pond that is open at the top and comprises a cultivating medium 21 in which algae are propagated or breed. The cultivating medium 21 contacts the environment such that a gas (for example oxygen) or a gaseous composition can directly emitted into the environment. Furthermore, the cultivation medium 21 can be illuminated or irradiated by natural or artificial light L from the above. The amount of light 21, which can be absorbed by the cultivating medium 21, depends on the accessible area of the medium 21 (level surface 22). Carbon dioxide that is needed for maintaining life sustaining processes and particularly for growth is provided by a carbon dioxide supply R, wherein the carbon dioxide is transported via a carbon dioxide supply line 13 into the cultivation medium 21 and discharged by a multitude of perforated hoses 12 that are e.g. designed as a gas diffuser mat 11. The perforated hoses 12 are made of or comprise a chemical resistant elastomer, particularly an ethylene-propylene elastomer or a styrene-butadiene elastomer. The dosage of the carbon dioxide is controlled by monitoring of the pH of the cultivation medium 21 by means of a pH meter 31 connected to a pH electrode 32, wherein the pH electrode protrudes into the medium 21. Other means for dosage control may also be used.

According to the invention, it is envisaged that carbon dioxide is guided through the multitude of perforated tubes with an excess pressure of 0.1 to 1 bar with respect to the pressure of the cultivation medium and a gas flow of 0.05 to 0.2 kg CO₂/(m*h). At the given pressure and gas flow, the pores of the perforated hoses 12 open and small bubbles of carbon dioxide are emitted into the cultivation medium but stay for a certain period of time at the hoses 12. Advantageously, such dwelling of the gas bubbles improves the efficiency of solving carbon dioxide in the cultivation medium. Moreover, oxygen that is produced by the bred algae can enter these gas bubbles resulting in the formation of oxygen enriched gas bubbles. After said period of time, the oxygen enriched gas bubbles detach from the hose, float to the surface of the cultivation medium and are emitted into the environment. By this, oxygen can advantageously be stripped of the cultivation medium 21.

A perforated hose that is particularly suitable for practicing the method of the invention is characterized by the following specifications:
- Material: ethylene-propylene diene rubber (EPDM) or styrene-butadiene rubber (SBR)
- Structure: two layers with textile insert (improved mechanical stability
- Perforation: 0.4 mm to 0.65 mm; 1000 - 3000 pores/m (pores per meter of the hose)
- Inner diameter: 10 mm to 19 mm
- Wall thickness: 3 mm to 4.5 mm

Such a perforated hose as described above can be used in an aqueous medium ranging from fresh water to very salted water.

**Reference numerals**

| | |
|---|---|
| R | carbon dioxide supply |
| P | breeding pond/basin |
| L | light |
| 11 | gas diffuser mat |
| 12 | perforated hose |
| 13 | carbon dioxide supply line |
| 14 | carbon dioxide comprising gas stream |
| 21 | breeding/cultivating medium |
| 22 | level surface of the breeding/cultivating medium |
| 31 | pH meter |
| 32 | pH electrode |

## Claims

1. Method for solving carbon dioxide in an aqueous medium (21) comprised within a tank (P), wherein gaseous carbon dioxide is discharged into said aqueous medium (21) via at least one perforated hose (12),
**characterized in that**
said carbon dioxide is guided through said at least one perforated hose (12) with a gas flow of 0.05 to 0.2 kg CO₂/(m*h).

2. Method according to claim 1, **characterized in that** said carbon dioxide is guided through said at least one hose (12) with an excess pressure of 0.1 to 1 bar with respect to the pressure of the aqueous medium (21).

3. The method according to claim 1 or 2, **characterized in that** said at least one perforated hose (12) is at least in part arranged at the bottom of said tank (P).

4. The method according to one of claims 1 to 3, **characterized in that** said hose is **characterized by** 1000 to 3000 pores per meter of the hose, particularly with a pore size of 0.40 mm to 0.65 mm.

5. The method according to one of claim 1 to 4, **characterized in that** said hose is made of an elastomer, particularly an ethylene-propylene elastomer or styrene-butadiene elastomer.

6. The method according to one of claims 1 to 5, **characterized in that** said tank (P) is **characterized by** a fill level of 0.1 m to 0.4 m.

7. The method according to one of claims 1 to 6, **characterized in that** said at least one perforated hose (12) is designed as a gas diffuser mat (11).

8. The method according to any one of claims 1 to 7, **characterized in that** said tank (P) is used for propagating a carbon dioxide assimilating organism, particularly an algae or a cyanobacterium.

9. The method according to any one of claims 1 to 8, **characterized in that** said tank (P) is an open pond.

10. The method for propagating an autotrophic carbon dioxide assimilating organism, the method comprising the steps of:
- providing a tank (P) comprising an aqueous medium (21) and an autotrophic carbon dioxide assimilating organism,
- discharging a gaseous stream comprising carbon dioxide into said aqueous medium (21), and
- cultivating said autotrophic carbon dioxide assimilating organism,
**characterized in that**
said gaseous stream is discharged into said aqueous medium by a method according one of claims 1 to 9.

11. The method according to claim 10, **characterized in that** said carbon dioxide assimilating organism is an algae or cyanobacterium.

12. The method according to claim 10 or 11, **characterized in that** said tank (P) is an open pond.
